# EUROPEAN PATENT SPECIFICATION

(11) **EP 0 833 620 B1**
(45) Date of publication and mention of the grant of the patent: **09.10.2002**
(21) Application number: 96917640.3
(22) Date of filing: 12.06.1996
(51) Int. Cl.: A61K 9/22, A61K 39/395, C07K 16/00, C12P 21/08, C12N 5/00, G01N 33/558

(54) **FGFR3 AS A MARKER FOR MESENCHYMAL SKELETAL PROGENITOR CELLS**
FGFR3 ALS SIGNAL FÜR DIE VORLÄUFER DER MESENCHYMZELLEN DES SKELETTS
FGFR3 UTILISE EN QUALITE DE MARQUEUR DE CELLULES PROGENITRICES MESENCHYMATEUSES DU SQUELETTE

(30) Priority: 12.06.1995 US 60137
(43) Date of publication of application: 08.04.1998
(73) Proprietor: YEDA RESEARCH AND DEVELOPMENT CO. LTD., Rehovot 76100 (IL); RAMOT UNIVERSITY AUTHORITY FOR APPLIED RESEARCH & INDUSTRIAL DEVELOPMENT LTD., Tel Aviv 69975 (IL)
(72) Inventor: YAYON, Avner, . (IL); NEVO, Zvi, 46425 Herzlia (IL)
(74) Representative: Vossius, Volker, Dr.
(86) International application number: PCT/IL96/00010
(87) International publication number: WO 96/041620

(56) References cited:
- US-A- 5 226 914
- K. PETERS ET AL.: "Unique expression pattern of the FGF receptor 3 gene during mouse organogenesis." DEVELOPMENTAL BIOLOGY, vol. 155, no. 2, February 1993, SAN DIEGO, CA, USA, pages 423-430, XP002056194
- K. KEEGAN: "Identification and characterization of an additional member of the fibroblast growth factor receptor family, FGFR-3 (leukemia cell line K562)." DISSERTATION ABSTRACTS INTERNATIONAL. B. THE SCIENCE AND ENGINEERING, vol. 52, no. 9, 1992, page 4594 XP002056195
- D. HECHT ET AL.: "Identification of fibroblast growth factor 9 (FGF9) as a high affinity, heparin dependent ligand for FGF receptors 3 and 2 but not for FGF receptors 1 and 4." GROWTH FACTORS, vol. 12, no. 3, 1995, LONDON, GB, pages 223-233, XP002056196
- S. MCLESKEY ET AL.: "MDA-MB-134 breast carcinoma cells overexpress fibroblast growth factor (FGF) receptors and are growth-inhibited by FGF ligands." CANCER RESEARCH, vol. 54, no. 2, 15 January 1994, BALTIMORE, MD, USA, pages 523-530, XP002056198
- NATURE, 15 September 1994, Vol. 371, ROUSSEAU et al., "Mutations in the Gene Encoding Fibroblast Growth Factor Receptor-3 in Achondroplasia", pages 252-254.
- EUR. J. PEDIATR., March 1995, Vol. 154, SUPERTI-FURGA et al., "A Glycine 375-to-Cysteine Substitution in the Transmembrane Domain of the Fibroblast Growth Factor Receptor-3 in a Newborn with Achondroplasia", pages 215-219.
- CELL, 22 March 1996, Vol. 84, DENG et al., "Fibroblast Growth Factor Receptor 3 is a Negative Regulator of Bone Growth", pages 911-921.
- DIFFERENTIATION, 1994, Vol. 58, LAUNAY et al., "Comparative Analysis of the Tissue Distribution of Three Fibroblast Growth Factor Receptor mRNAs During Amphibian Morphogenesis", pages 101-111.
- IWAMOTO M ET AL.: 'Reduction in Basic Fibroblast Growth Factor Receptor is coupled with terminal differentiation of chondrocytes' J. BIOL. CHEM. vol. 266, no. 1, 05 January 1991, pages 461 - 467
- SASSE J et al. "Expression of Fibroblast Growth Factor Receptors During Cartilage Differentiation", poster published on 27.-29.04.2000
- NEVO Z et al. "The Manipulated Mesenchymal Stem Cells in Regenerated Skeletal Muscles", Cell Transplantantion, Vol. 7, Nr. 1 63-70, 1998
- ROBINSON D et al. "Fibroblast Growth Factor Receptor-3 as a Marker for Precartilaginous Stem Cells", Clin. Orthopaedics and Related Research, Nr. 3673, S163-S175, 1999

## Description

### FIELD OF THE INVENTION

The present invention concerns a method for identifying mesenchymal skeletal progenitor cells by identification of cells which feature on their surface fibroblast growth factor receptor 3 (FGFR3).

The present invention further concerns a method for obtaining mesenchymal skeletal progenitor cells by utilizing FGFR3-binding agents. The invention still further concerns a substantially pure culture of mesenchymal skeletal progenitor cells as well as pharmaceutical compositions and implants comprising said mesenchymal skeletal progenitor cells.

By another aspect the invention concerns a method for identification of cartilage-bony tumor and pharmaceutical compositions for the treatment of cartilage-bony tumor.

### BACKGROUND OF THE INVENTION

Skeletal growth depends both on proper function of the tissue cellular elements - the chondrocytes, and their cell membrane receptors in the cartilaginous growth centers of the long bones, as well as on the normalcy and levels of circulating and local hormones and growth factors. Growth disorders are therefore classified into two distinct categories (a) failures in a circulating factor, and (b) failures in the target cartilaginous tissue.

The course of normal differentiation begins with mesenchymal stem cells which differentiate to skeletal progenitor cells which can either differentiate to precartilaginous stem cells, which eventually form the cartilage, or to preosteogenic stem cells which eventually form the bone.

Marrow derived mesenchymal cells which can be activated to differentiate into specific types of connective tissue, and which can be used to treat skeletal and other connective tissue disorders have been described in US 5,226,914.

In attempts to trace the mesenchymal stem cells supporting growth and their routes of migration in normals and in the family of growth disorders, there are difficulties, including the lack of proper markers for these specific mesenchymal stem cells. For example, spotty and incomplete information is available regarding the original location and the routes of migration of the growth plate stem cells, supporting the longitudinal and the transverse growth. A long lasting dispute of over a hundred years, which may be called *"Ranvier versus La Cro2ix"* is still perpetuating. In 1889 Ranvier stated *"Cells forming the periosteal bone, originate from the cells of the growth plate"*, while in 1951 La Croix declared *"Appositional growth occurs from cells of the peri-chondral periphery".* Ranvier's theory gained support at the early seventies from Rigal, Hert, J. (*Acta Anat (Bazel)* **82**:420-436 (1972)) and others, and in the nineties by Langenskiold *et al*. (Acta. Orthop. Scand., **64**:683-687 (1993)), suggesting that cells from the germinal layer migrate to the borderline of the bone groove, serving as the source for both longitudinal and transverse bone growth.

A full understanding of the various types of cartilage cells and the factors that effect mesenchymal differentiation, however, has been hampered due to failure to locate the original location of the primary reservoir of these cells and thus the limitations of *in vitro* cell culture. One difficulty has been the lack of specific phenotypic markers to follow successive differentiation events. Type II collagen secretion is considered a major early marker of chondrocyte differentiation, while the synthesis of alkaline phosphatase is an early marker of osteoblast differentiation. Mature osteoblasts also produce osteopontin, osteonectin, and osteocalcin, three extracellular matrix proteins deposited together with type I collagen into mineralized bone matrix. Unfortunately, only a few differentiation markers have been identified, and several of these, such as alkaline phosphatase, osteopontin, and osteonectin, are not specific for osteogenic differentiation, while others, such as osteocalcin, are rarely expressed *in vitro.* In addition, mesenchymal cell lines and primary cultures of differentiating chondrocytes and osteoblasts display a variable phenotype and are often a mixture of cell types at different stages of differentiation (Eriebacher, A. *et al, Cell* **80**:371-378, (1995); Yamaguchi, T.P. and Rossant, J., *Current Opinion in Genetics and Development* **5**:485-491 (1995)).

Thus it would have been highly desirable to develop a marker capable of locating precisely the site and source of stem cells supporting and contributing to both longitudinal and transverse growth and for bone repair both for better understanding of the mechanism of mesenchymal development in normal and pathological conditions, as well as for the purpose of obtaining a substantially pure culture of mesenchymal skeletal progenitor cells for therapeutical purposes.

### SUMMARY OF THE INVENTION

The present invention is based on the surprising finding that fibroblast growth factor receptor 3 (FGFR3) serves as a marker for mesenchymal skeletal progenitor cells. The present invention is further based on the surprising finding that the anatomical location of mesenchymal skeletal progenitor cells is in the perichondrium in the La Croix groove.

The term *"mesenchymal skeletal progenitor cells"* will be used in the following to denote the following types of cells: (a) mesenchymal stem cells which are able to differentiate to skeletal progenitor cells, (b) skeletal progenitor cells, (c) precartilaginous stem cells, and (d) preosteogenic stem cells or a combination of two or more of the above cell types. The mesenchymal skeletal progenitor cells all share the property of contribution to the growth of bone and/or cartilage, show enhanced proliferation properties as compared to other types of cartilage and bone derived cells and also a tendency to migrate in the presence of suitable chemotactic agents such as fibroblast growth factor 9.

These mesenchymal skeletal progenitor cells, in early stages of embryonal and neonatal life, support the growth of both articular and physis growth-plate cartilages. However, quite early in life, a few months post-birth, the connection of these stem cells to the articular zone is abolished leading to the poor self-wound healing of articular cartilage. Such mesenchymal skeletal progenitor cells continue to maintain the cell source for the longitudinal and latitudinal (transverse) growth of long bones, until the closure of the physis (at the age of 18-22 years), and continue to provide the stem cell reservoir of the periosteum, involved in the callous of bone fractures all through life. In adult life, especially at advanced ages, a technique for tracing undifferentiated cell source with a potential to establish proliferating chondrocytes has previously failed due to the scarcity of such a cell source and the inadequatabilily of markers for such undifferentiated cells.

By using the discovery on which the present invention is based, namely that FGFR3 is a marker for mesenchymal skeletal progenitor cells, it was possible to develop a method for identification of mesenchymal skeletal progenitor cells by identifying those cells which feature FGFR3 on their surface. Such a method may be important for tracing mesenchymal skeletal progenitor cells for example for better understanding of pathological conditions of growth arrest involving FGFR3 receptors for example those leading to genetic dwarfism-achondroplasia or persistent expression in multiple hereditary exostosis and reexpression in primary osteoarthritic osteophytes.

Thus the present invention provides a method for identifying mesenchymal skeletal progenitor cells comprising:
**(a)** applying a fibroblast growth factor receptor 3 (FGFR3) binding agent to assayed cells or tissue under conditions allowing ligand-receptor binding;
**(b)** determining which cells bound said FGFR3 binding agent, said cells being mesenchymal skeletal progenitor cells.

The FGFR3 binding agent which may be an antibody or fibroblast growth factor 9 (FGF9) should be labeled and applied to the assayed tissue, for example to tissue of the joint. Those regions which are labeled serve as a source for mesenchymal skeletal progenitor cells.

Preferably, the source for the mesenchymal skeletal progenitor cells is the perichondrium at the region of La Croix, and the region which meets the synovial membrane and the periosteum.

The method of the present invention may be used to identify and locate mesenchymal skeletal progenitor cells in various tissues such as at the joints for various purposes, for example for obtaining a culture of mesenchymal skeletal progenitor cells, proliferating them *in vitro* and then reintroducing them to the body in order to encourage cartilage and bone growth. Alternatively, identifying these cells enables their removal from the tissue site in order to eliminate excess activity of such stem cells in various diseases and disorders characterized by over-activity of undifferentiated mesenchymal skeletal progenitor cells. Furthermore, by locating the regions of the FGFR3 carrying cells, it is possible to manipulate such cells *in situ* by administering to the exact location of these cells various modulating agents. Such agents may be agents capable of stabilizing the FGFR3 and thus maintaining for longer periods of time the undifferentiated proliferating state of these cells, an example being FGF9. Alternatively, the agent may cause premature differentiation of FGFR3 carrying cells, an example being an FGF9 antagonist.

By using the fact that FGFR3 is a marker of mesenchymal skeletal progenitor cells, it was possible for the first time to obtain a substantially pure culture of such cells from a non-embryonic source. According to the method of the present invention, by using the FGFR3 as a marker, it was found that the mesenchymal skeletal progenitor cells are located in the perichondrium ring (region of La Croix) present in the periphery of the growth plates. The poor self-wound healing of articular cartilage late in life may be explained on the basis of disconnection of these articular zones from the source of their potential stem cells at the perichondrial La Croix region which occurs at the cessation of growth.

Thus, the present invention enables not only localization of mesenchymal skeletal progenitor cells, but also obtaining for the first time a substantially pure culture of large amounts of such cells. The term *"substantially pure culture"* denotes a culture composed essentially of one or more of the four cell types covered by the term *"mesenchymal skeletal progenitor cells"* as defined above.

The present invention thus concerns a method for obtaining large amounts of mesenchymal skeletal progenitor cells from various sources, as will be explained hereinbelow. The mesenchymal skeletal progenitor cells may be identified, and separated from the other cells in the source, by utilizing either specific antibodies against FGFR3, or by using a specific ligand for this receptor such as the FGF9 ligand.

The method for obtaining a substantially pure culture of mesenchymal skeletal progenitor cells comprises:
**(c)** applying an FGFR3-binding agent to a cell source containing mesenchymal skeletal progenitor cells; and
**(d)** separating from said source only cells which are bound FGFR3, said cells providing a substantially pure culture of mesenchymal skeletal progenitor cells.

Separation may be carried out surgically, for example by picking up with a scalpel only those regions which are bound to an FGFR3 labeled binding agent, or may be carried out by utilizing various cell separation systems which are able to separate individual cells bearing a specific label (the FGFR3 binding agents) from an unlabeled population of cells in the source.

Suitable sources for obtaining such mesenchymal skeletal progenitor cells is an autogenic source available from arthroscopic or bone marrow biopsies. The biopsy source may be non-proliferating chondrocytes or dedifferentiated fibroblast-like cells. The cell source may also be obtained from regions of the perichondrium, synovial membrane or periosteum or the location in which these regions meet. Only by utilizing a specific marker it is possible to isolate mesenchymal skeletal progenitor cells from these sources due to the scarcity of these cells in the tissue. Alternatively the cell source may also be embryonic.

Mesenchymal skeletal progenitor cells obtained from these sources may be induced to proliferate *ex vivo* in the presence of suitable growth factors and heparin and then reintroduced into the body either in the form of pharmaceutical composition within a medium suitable for maintaining the viability of chondrocytes, or introduced to the desired site in the form of an implant, wherein the mesenchymal skeletal progenitor cells are present inside a growth-permissive gluey milieu. It is preferable that both the pharmaceutical composition and the implant contain also suitable fibroblast growth factor, preferably fibroblast growth factor 9, in order to stimulate the activity of the FGFR3 present on those mesenchymal skeletal progenitor cells.

The pharmaceutical compositions or the implant of the invention may be used for the purpose of repair and regeneration of defective articular cartilage, for treatment of achondroplastic patients, for treatment of patients suffering from other growth disturbances and for treatment of physical injuries with poor predicted rate of cartilage and bone growth. The pharmaceutical composition or the implant of the invention may be used as interventions for manipulating the rate of growth within growth plates in order to increase the growth rate and/or prevent premature differentiation; or may be used for direct injection into the nucleus pulposus of the fine vertebrae in order to enhance the healing of spine injuries. If desired, the autologous mesenchymal skeletal progenitor cells may be altered, *ex vivo* by molecular engineering to express desirable traits prior to introduction into the desired site. Examples of genetic manipulations are those directed to over-expression of wild type FGFR3 in order to replace a mutant defective receptor, or the expression of a dominant negative mutant FGFR3 in order to suppress the activity of a wild type receptor, for example, in the cases of various types of tumors and the like.

In practice, the method of the invention comprises embedding the mesenchymal skeletal progenitor cells in a viscous growth-permissive milieu, usually based on hyaluronic acid, forming a composite semi-solid implant. The implant is transferred to the target site of growth, for example the articular lesion site, either under open joint surgery or by an arthroscopic device, filling the lumen of the injury to the articular surface. A thin permeable film is formed by a spraying device, closing the defect and ensuring the anchorage and maintenance of the implant in its authentic place.

By another aspect, the present invention is based on the finding that FGFR3 is also present on cartilagineous-bony tumors, for example, benign tumors (e.g. exostosis and osteophytes) and thus may serve both as an indicator of the presence of such a tumor as well as a marker for the precise localization of such tumors. Therefore, the present invention further comprises a method for detection of cartilaginous-bony tumors in a tissue or a sample comprising:
(i) contacting the assayed tissue or sample with an FGFR3 binding agent;
(ii) detecting the presence of cells which bound FGFR3 binding agent a positive detection indicating the presence of a cartilaginous-bony tumor in the assayed tissue or sample.

The detection may be carried out by using suitable labeled antibodies against FGFR3, or by use of specific labeled ligands for FGFR3, such as FGF9. By applying said labeled FGFR3 binding agent to a tissue *in vivo* it is possible not only to determine whether a tumor is present in the tissue, but also to precisely localize the tumor which may help in surgical removal thereof.

The fact that the FGFR3 is present on cartilaginous-bony tumor cells, may also serve to target cytotoxic agents specifically to the site of the tumor, by attaching to a specific ligand to FGFR3 such as FGF9, or a specific antibody against FGF9, a suitable cytotoxic moiety. Thus the present invention is further directed to pharmaceutical compositions for the treatment of cartilaginous-bony tumors comprising an FGFR3 binding agent attached to a cytotoxic moiety, as well as to a method for the treatment of such tumors by administering to a subject a therapeutically effective amount of FGFR3 attached to a cytotoxic moiety.

Cytotoxic agents are well known in the art and this term, within the context of the present invention, refers to any agent capable of destroying cartilage- and bone-derived tumor cells. Examples of such agents are, for example methotrexate, doxombicin, cyclophosphamides. etc.

Treatment of cancer may also be carried out by inducing differentiation of FGFR3 carrying cells. This may be carried out for example by introduction of FGFR3 differentiation inducing agents to regions labeled by a FGFR3-binding agent. Examples of differentiation inducing agents are FGF9 antagonists or antibodies against FGF9.

The treatment may also be carried out by introducing to the tumor a dominant negative detective FGFR3 (for example, by genetic engineering) which attenuates the activity of the wild type FGFR3.

In the following the invention will be illustrated with reference to some non-limiting drawings and examples.

### DETAILED DESCRIPTION OF THE DRAWINGS

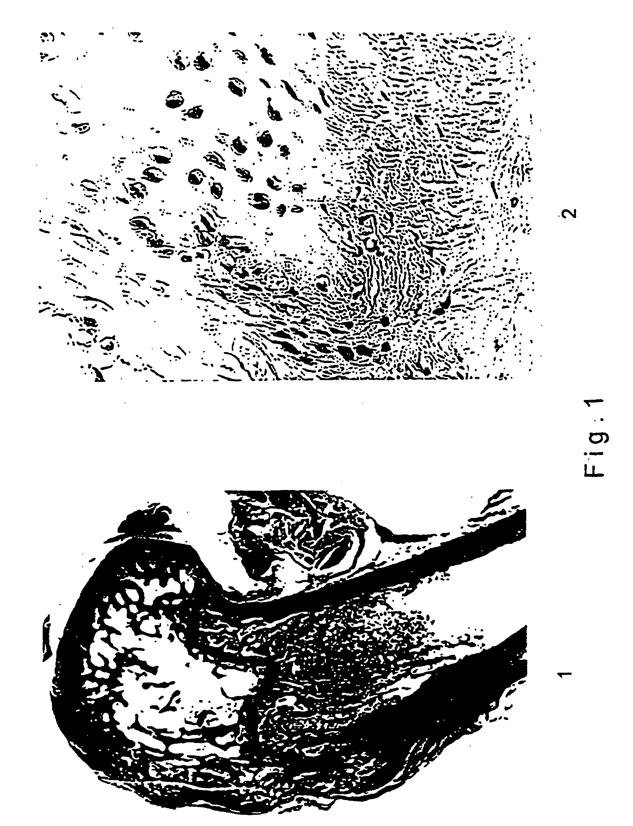

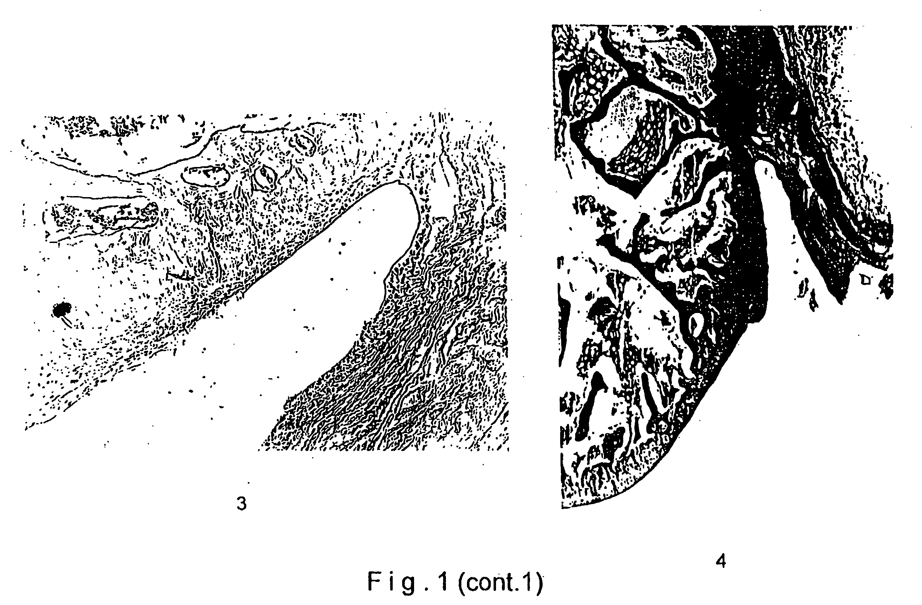

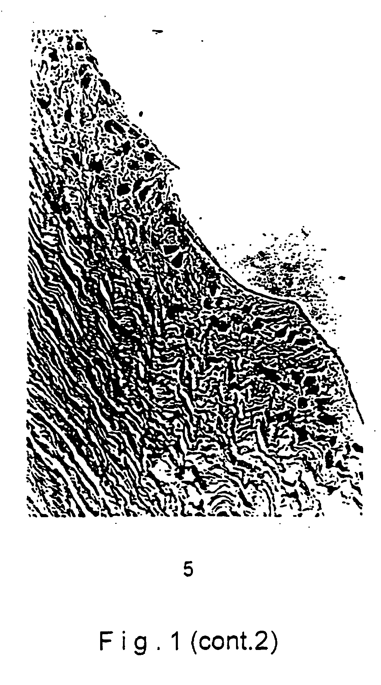

**Fig. 1** - Histological staining of epiphyseal section of guinea pig by antibodies against FGFR3. Photo-micrographs 1-5 represent sections through the epiphyses of young adult guinea pig.
   1. Sagittal section stained with the histological dye of Masson's trichrome (magnification x 6) which is a specific staining for connective tissue elements such as collagen and protoglycans.
   2. Sagittal section stained by immunohistochemical staining with antibodies against FGFR3 ( x 400). 3, 4 & 5. Axial sections.
   3. Immunohistochemical staining for the antibody agent FGFR3 (x 100);
   4. Masson's trichrome (x 25); and
   5. Immunohistochemical staining for the antibody agent FGFR3 (x 400).

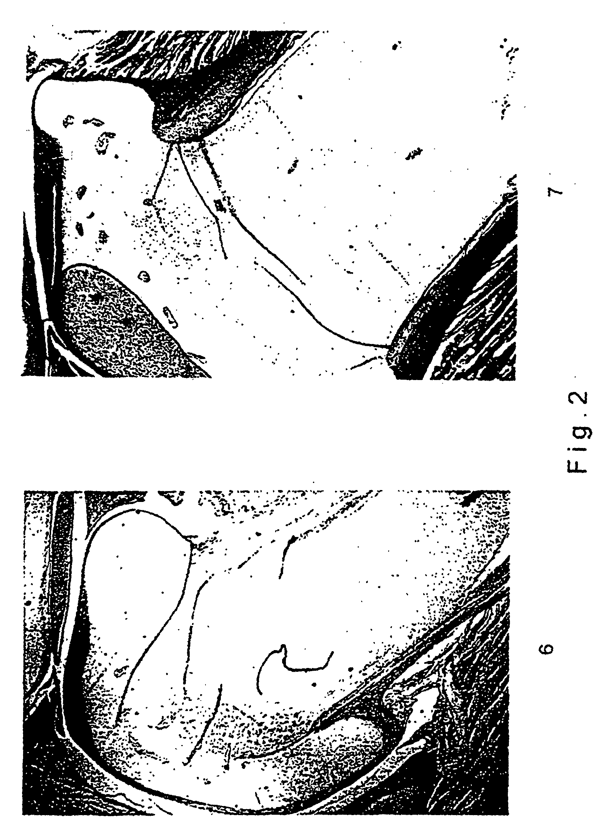

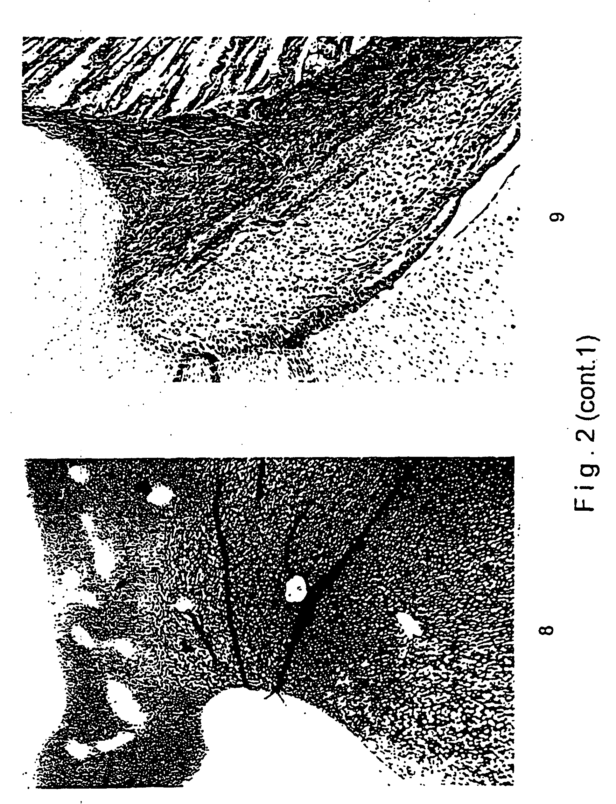

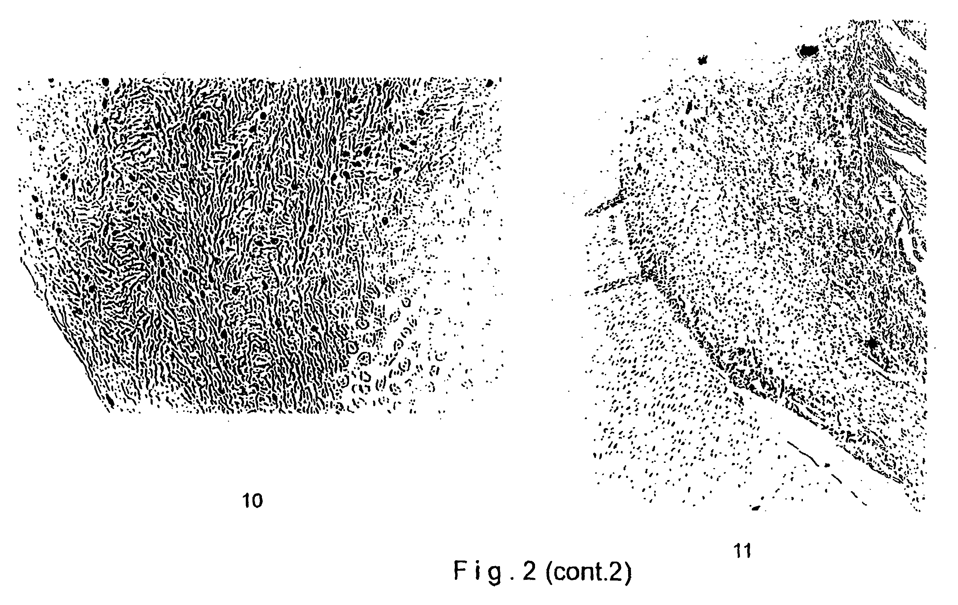

**Fig. 2** consists of photomicrographs 6-11 representing sagittal sections through the epiphyses of 17 days old chick embryos of long bones.
   Photomicrographs 6, 10 & 11 are stained by the immunohistochemical staining with antibodies against FGFR3 and 8 is stained by Alcian blue pH 2.5 specific for protoglycans (note lack of staining in certain areas).
   The photomicrographs are magnified as follows: 6 (x 25); 7 (x 40); 9 (x 100); 10 (x 400); and 11 (x 100).
   7 & 9 are stained by Masson's trichrome.
**Fig. 3** shows the femur growth in adolescent rats in which the prechondrian ring surrounding the physis was removed (STRIP); in rats which underwent exposure of perichondrium without its removal (SHAM); and in rats which did not undergo any operation (CNTL); and
**Fig. 4** shows the number of days until colony formation of cells, grown *in vitro,* obtained from articular cartilage, epiphysis, physis and perichondrium.

### DETAILED DESCRIPTION OF THE INVENTION

### Materials and Methods

### (a) Primary Chondrocyte Culture:

Epiphysis of long bones (femur and tibia) were obtained from 11 days old chicken embryos. After dissection, tissue segments were treated with trypsin in Tyrod's solution and mechanically disrupted until free cell suspension was obtained. Cells were then plated in high concentration (5x10⁶).

### (b) PCR screening of primary chondrocyte cultures:

When confluence was reached, cells were collected and lysed by RNA purification kit (tri-reagent) (Molecular Research Center, Cincinnati, Ohio). RNA from cells was phenol extracted, isopropanol precipitated, resuspended in water, and assayed by measuring its optic density. After obtaining clean RNA (O.D. 260/280nm.>1.5), cDNA using reverse transcriptase reaction was made and screened for fibroblast growth factors (FGFs). The polymerase chain reaction (PCR) technique was used, employing oligonucleotide pairs for both FGFR3 and FGF9. Denaturation was at 94°C, annealing at 52-65°C, and elongation at 72°C, repeated for 35 cycles.

### (c) Radiolabeling of FGF9:

Recombinant mouse FGF9 was prepared as previously described (Hecht, D. *et al*, Growth factors **12**, 223-233 (1995)) labeled with Na¹²⁵I (0.5mCi) using the Chloramine-T method and separated from free iodine on a heparin-sepharose column. The range of specific activity was 0.5-2 x 105 cpm/ng.

### (d) Immunohistochemistry:

Decalcified bones were embedded in liquid paraffin after fixation by formalin and picric acid. Paraffin blocks were cut and prepared for immunohistochemistry using a standard protocol. Staining of slides was done with ascending titer of anti-FGFR3 antibody.

### (e) In situ hybridization:

T7 (antisense probe) and T3 (sense probe) were made from recombinant FGF9 and FGFR3 containing plasmid (Bluescript-Stratagen), using S-35 labeled uridine residues. Mouse embryos aged 10.5 to 18.5 days post-conception were fixed in paraformaldehyde, dehydrated in ascending concentrations of ethanol, and embedded in liquid paraffin. Sections were cut and prepared and hybridized with a proper RNA probe by standard methods.

### Example 1

### Histochemical staining by antibodies against FGFR3

As can be seen in Fig. 1, regions which were stained with antibodies against FGFR3 did not correspond to regions stained by Masson's trichrome which is an accepted cartilage stain. These findings indicate that FGFR3 bearing cells are not located in the cartilage itself but rather in the perichondrium in the region known as La Croix groove.

### Example 2

### Stripping of the ring of La Croix in adolescent rats

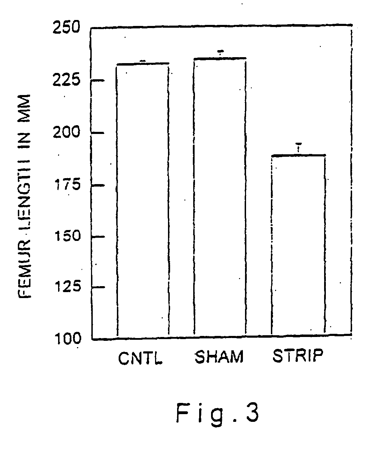

10 rats were included in each of three groups. Group 1 served a s a control group (CNTL). Rats were anesthetized but no operation was performed. Group 2 (SHAM) served as a sham operation group and underwent anesthesia and dissection of soft tissues exposing the perichondrium. Group 3 (STRIP) underwent stripping of the perichondrial ring surrounding the physis under loop magnification which allowed dissection of the soft tissues only without any damage to the physis itself. 4 weeks later average femur length was measured in rats and the results are shown in Fig. 3. The contra-lateral limb was similar in length in operation to the control limbs (data not shown). The sham operated limbs demonstrated a tendency for increase in limb length which did not reach statistical significance. The stripped limbs demonstrated a growth arrest of the limb. These results indicated that removal of regions which were stained by FGFR3-antibodies causes arrest in limb length indicating the involvement of such regions in normal growth.

### Example 3

### In vitro growth of cells obtained from the ring of La Croix

Perichondrial tissue from the La Croix region removed from the above rats was placed in a culture dish in a suitable growth medium and the period until colony formation was determined. In comparison, tissue obtained from various locations of the distal femur (articular cartilage, epiphysis (bone), physis (cartilage)) was cultured under the same conditions and the period until colony formation was also determined.

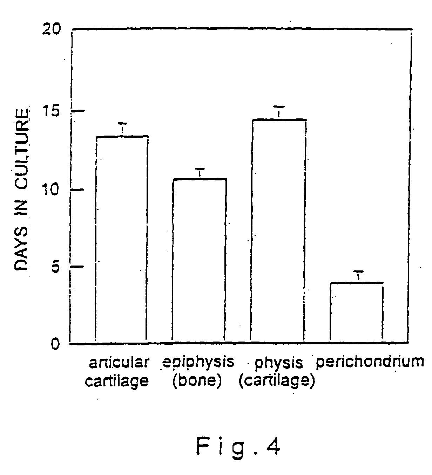

As can be seen in Fig. 4, tissue removed from the perichondrium demonstrated an ability to rapidly form cell colonies after about 3 days in culture, while tissue removed from other regions formed cultures only after more than ten days from implantation. These results again indicate that cells obtained from the region stained with FGFR3-antibodies grow more rapidly than cells obtained from other regions of the bone which do not feature FGFR3.

### Example 4

### Presence of FGFR3 in exostosis

Antibodies against FGFR3 were applied to tissue obtained from exostosis benign tumor. The antibodies stained cells in the fibrotic tissue and essentially did not stain cells of the cartilage (data not shown). These findings indicate that FGFR3 is present in cartilaginous-bony derived benign tumor (exostosis) so that FGFR3 binding agents (such as antibodies) may be used to identify such tumors as well as to target cytotoxic agents thereto. This finding also leads to the treatment of such tumors by agents which cause disappearance of FGFR3 (for example antagonist of FGF9) and thus lead to differentiation.

## Claims

1. A method for identifying mesenchymal skeletal progenitor cells comprising:
(a) applying a fibroblast growth factor receptor 3 (FGFR3)-binding agent to assayed cells or tissue under conditions allowing ligand-receptor binding;
(b) determining which cells bound said FGFR3-binding agent, said cells being mesenchymal skeletal progenitor cells.

2. A method according to claim 1, wherein the FGFR3-binding agents are antibodies specific fbr FGFR3.

3. A method according to claim 1, wherein the FGFR3-binding agent is fibroblast growth factor 9 (FGF9).

4. A method for obtaining a substantially pure culture of mesenchymal skeletal progenitor cells, wherein said culture is composed of one or more of the following four cell types: (a) mesenchymal stem cells which are able to differentiate to skeletal progenitor cells, (b) skeletal progenitor cells, (c) precartilaginous stem cells, and (d) preosteogenic stem cells, said method comprising:
(a) applying FGFR3-binding agent to a cell source containing mesenchymal skeletal progenitor cells; and
(b) separating from said source cells which bound said FGFR3-binding agent, said cells providing a substantially pure culture of mesenchymal skeletal progenitor cells.

5. A method according to claim 4, wherein the cell source is autogeneic being non-proliferating chondrocytes, dedifferentiated fibroblast-like cells, cells obtained from the perichondrium, synovial membrane, periosteum, or the cell source are non-human embryonic cells.

6. A substantially pure culture of mesenchymal skeletal progenitor cells which feature FGFR3 on their surface, wherein said culture is composed of one or more of the following four cell types: (a) mesenchymal stem cells which are able to differentiate to skeletal progenitor cells, (b) skeletal progenitor cells, (c) precartilaginous stem cells, and (d) preosteogenic stem cells said cells being identifiable by the method of any one of claims 1 to 3.

7. A substantially pure culture of mesenchymal skeletal progenitor cells according to claim 6, wherein said cells are derived from the perichondrium, synovial membrane or periosteum or the location in which these regions meet.

8. A substantially pure culture of mesenchymal skeletal progenitor cells according to claim 6, wherein said cells are precartilaginous stem cells.

9. A pharmaceutical composition suitable for the repair of bone and cartilage comprising the mesenchymal skeletal progenitor cells of any one of claims 6 to 8, and a medium suitable for maintaining the viability of chondrocytes.

10. A pharmaceutical composition according to claim 9 further comprising a member of the FGF family which binds FGFR3.

11. A pharmaceutical composition according to claim 10, comprising FGF9.

12. An implant suitable for bone or cartilage implantation comprising a growth permissive gluey milieu and mesenchymal skeletal progenitor cells of any one of claims 6 to 8.

13. An implant according to claim 12 further comprising a member of the FGF family which binds FGFR3.

14. An implant according to claim 13, comprising FGF9.

15. A method for detection of cartilaginous-bony tumors in vitro in a tissue or a sample comprising:
(a) contacting the assayed tissue or sample with an FGFR3 binding agent
(b) detecting the presence of cells which bind to said FGFR3 binding agent, a positive detection indicating the presence of a cartilaginous-bony tumor in the assayed tissue or sample.

16. The method according to claim 15, wherein the cartilaginous-bony tumor is benign and is exostosis or osteophyte.

## Patentansprüche

1. Ein Verfahren zur Identifizierung mesenchymaler skelettaler Vorläuferzellen, umfassend:
a) Anwendung eines Fibroblasten-Wachstumsfaktor-Rezeptor 3 (FGFR3)-bindenden Agens auf untersuchte Zellen oder Gewebe unter Bedingungen, die eine Liganden-Rezeptor-Bindung ermöglichen;
b) Bestimmung, welche Zellen das FGFR3-bindende Agens gebunden haben, wobei diese Zellen mesenchymale skelettale Vorläuferzellen sind.

2. Verfahren gemäß Anspruch 1, wobei die FGFR3-bindenden Agenzien für FGFR3 spezifische Antikörper sind.

3. Verfahren gemäß Anspruch 1, wobei das FGFR3-bindende Agens Fibroblasten-Wachstumsfaktor 9 (FGF 9) ist.

4. Ein Verfahren zum Erhalt einer im wesentlichen reinen Kultur mesenchymaler skelettaler Vorläuferzellen, wobei diese Kultur im wesentlichen aus einem oder mehreren der folgenden vier Zelltypen besteht: (a) mesenchymale Stammzellen, die zur Differenzierung zu skelettalen Vorläuferzellen fähig sind, (b) skelettale Vorläuferzellen, (c) präkartilaginäre Stammzellen, und (d) präosteogene Stammzellen, wobei dieses Verfahren umfasst:
(a) Anwendung eines FGFR3-bindenden Agens auf eine Zellquelle, die mesenchymale skelettale Vorläuferzellen enthält; und
(b) Abtrennung von Zellen, die das FGFR3-bindende Agens gebunden haben, aus dieser Quelle, wobei diese Zellen eine im wesentlichen reine Kultur mesenchymaler skelettaler Vorläuferzellen bereitstellen.

5. Verfahren gemäß Anspruch 4, wobei die Zellquelle autogen ist, wobei die Zellen nicht-proliferierende Chondrocyten, dedifferenzierte fibroblastenartige Zellen, aus dem Perichondrium, der Synovialmembran, dem Periost erhaltene Zellen sind, oder die Zellquelle sind nicht-menschliche embryonale Zellen.

6. Eine im wesentlichen reine Kultur mesenchymaler skelettaler Vorläuferzellen, die FGFR3 auf ihrer Oberfläche aufweisen, wobei diese Kultur im wesentlichen aus einem oder mehreren der folgenden vier Zelltypen besteht: (a) mesenchymale Stammzellen, die zur Differenzierung zu skelettalen Vorläuferzellen fähig sind, (b) skelettale Vorläuferzellen, (c) präkartilaginäre Stammzellen, und (d) präosteogene Stammzellen, wobei diese Zellen anhand des Verfahrens gemäß einem der Ansprüche 1 bis 3 identifizierbar sind.

7. Eine im wesentlichen reine Kultur mesenchymaler skelettaler Vorläuferzellen gemäß Anspruch 6, wobei diese Zellen vom Perichondrium, der Synovialmembran oder dem Periost oder der Stelle abgeleitet sind, in der sich diese drei Bereiche treffen.

8. Eine im wesentlichen reine Kultur mesenchymaler skelettaler Vorläuferzellen gemäß Anspruch 6, wobei diese Zellen präkartilaginäre Stammzellen sind.

9. Eine pharmazeutische Zusammensetzung, die zur Reparatur von Knochen und Knorpel geeignet ist, umfassend die mesenchymalen skelettalen Vorläuferzellen gemäß einem der Ansprüche 6 bis 8, und ein Medium, das zur Aufrechterhaltung der Lebensfähigkeit der Chondrocyten geeignet ist.

10. Eine pharmazeutische Zusammensetzung gemäß Anspruch 9, weiter umfassend ein Mitglied der FGF-Familie, das FGFR3 bindet.

11. Eine pharmazeutische Zusammensetzung gemäß Anspruch 10, umfassend FGF9.

12. Ein Implantat, das zur Knochen- oder Knorpel-Implantation geeignet ist, umfassend ein Wachstums-permissives klebriges Milieu und mesenchymale skelettale Vorläuferzellen gemäß einem der Ansprüche 6 bis 8.

13. Ein Implantat gemäß Anspruch 12, weiter umfassend ein Mitglied der FGF-Familie, das FGFR3 bindet.

14. Ein Implantat gemäß Anspruch 13, umfassend FGF9.

15. Ein Verfahren zum Nachweis knorpelig-knochiger Tumoren *in vitro* in einem Gewebe oder einer Probe, umfassend:
(a) In Kontakt bringen des untersuchten Gewebes oder der untersuchten Probe mit einem FGFR3-bindenden Agens
(b) Nachweis der Anwesenheit von Zellen, die an das FGFR3-bindende Agens binden, wobei ein positiver Nachweis die Anwesenheit eines knorpelig-knochigen Tumors in dem untersuchten Gewebe oder der untersuchten Probe anzeigt.

16. Verfahren gemäß Anspruch 15, wobei der knorpelig-knochige Tumor gutartig und Exostose oder Osteophyt ist.

## Revendications

1. Procédé pour l'identification de cellules de progéniteur de squelette de mésenchyme, comprenant:
(a) l'application d'un agent de liaison à un récepteur de facteur de croissance de fibroblastes 3 (FGFR3) pour tester des cellules ou tissus dans des conditions permettant la liaison ligand-récepteur;
(b) la détermination des cellules qui se sont liées au dit agent de liaison au FGFR3, lesdites cellules étant des cellules de progéniteur de squelette de mésenchyme.

2. Procédé selon la revendication 1, dans lequel les agents de liaison au FGFR3 sont des anticorps spécifiques pour FGFR3.

3. Procédé selon la revendication 1, dans lequel l'agent de liaison au FGFR3 est un facteur de croissance des fibroblastes 9 (FGF9).

4. Procédé pour l'obtention d'une culture substantiellement pure de cellules de progéniteur de squelette de mésenchyme, dans lequel ladite culture est essentiellement composée de l'un ou plusieurs des quatre types de cellules suivants: (a) cellules souches de mésenchyme qui sont capables de se différencier en cellules de progéniteur de squelette, (b) cellules de progéniteur de squelette, (c) cellules souches précartilagineuses, et (d) cellules souches préostéogéniques, ledit procédé comprenant:
(a) l'application d'un agent de liaison au FGFR3 à une source de cellules contenant des cellules de progéniteur de squelette de mésenchyme; et
(b) la séparation de ladite source des cellules qui se sont liées au dit agent de liaison au FGFR3, lesdites cellules procurant une culture substantiellement pure de cellules de progéniteur de squelette de mésenchyme.

5. Procédé selon la revendication 4, dans lequel la source de cellules est autogène, étant des chondrocytes non-prolifératifs, des cellules de type fibroblaste dédifférenciées, des cellules obtenues à partir de périchondre, de membrane synoviale, de périoste, ou la source de cellules sont des cellules embryonnaires non-mammaliennes.

6. Culture substantiellement pure de cellules de progéniteur de squelette de mésenchyme qui présentent du FGFR3 sur leur surface, dans laquelle ladite culture est essentiellement composée de l'un ou plusieurs des quatre types de cellules suivants: (a) cellules souches de mésenchyme qui sont capables de se différencier en cellules de progéniteur de squelette, (b) cellules de progéniteur de squelette, (c) cellules souches précartilagineuses, et (d) cellules souches préostéogéniques, lesdites cellules étant identifiables par le procédé selon l'une quelconque des revendications 1 à 3.

7. Culture substantiellement pure de cellules de progéniteur de squelette de mésenchyme selon la revendication 6, dans laquelle lesdites cellules sont dérivées du périchondre, de membrane synoviale ou de périoste, ou de l'emplacement où ces régions se rejoignent.

8. Culture substantiellement pure de cellules de progéniteur de squelette de mésenchyme selon la revendication 6, dans laquelle lesdites cellules sont des cellules souches précartilagineuses.

9. Composition pharmaceutique appropriée pour la réparation d'os et de cartilage, comprenant les cellules de progéniteur de squelette de mésenchyme selon l'une quelconque des revendications 6 à 8, et un milieu approprié pour le maintien de la viabilité de chondrocytes.

10. Composition pharmaceutique selon la revendication 9, comprenant en outre un membre de la famille FGF qui se lie au FGFR3.

11. Composition pharmaceutique selon la revendication 10, comprenant FGF9.

12. Implant approprié pour l'implantation d'os ou de cartilage, comprenant un milieu gluant permissif de croissance et des cellules de progéniteur de squelette de mésenchyme selon l'une quelconque des revendications 6 à 8.

13. Implant selon la revendication 12, comprenant en outre un membre de la famille FGF qui se lie au FGFR3.

14. Implant selon la revendication 13, comprenant FGF9.

15. Procédé pour la détection de tumeurs cartilagineuses-osseuses in vitro dans un tissu ou un échantillon, comprenant:
(a) la mise en contact du tissu ou de l'échantillon de test avec un agent de liaison au FGFR3
(b) la détection de la présence de cellules qui se lient au dit agent de liaison au FGFR3, une détection positive indiquant la présence d'une tumeur cartilagineuse-osseuse dans le tissu ou l'échantillon testé.

16. Procédé selon la revendication 15, dans lequel la tumeur cartilagineuse-osseuse est bénigne et est l'exostose ou l'ostéophyte.
